# EUROPÄISCHE PATENTSCHRIFT

(11) **EP 1 056 673 B1**
(45) Veröffentlichungstag und Bekanntmachung des Hinweises auf die Patenterteilung: **27.08.2003**
(21) Anmeldenummer: 99910164.5
(22) Anmeldetag: 29.01.1999
(51) Int. Cl.: B65H 39/16

(54) **VERFAHREN UND VORRICHTUNG ZUM EINBRINGEN EINER VIELZAHL VEREINZELTER FOLIENFÖRMIGER DARREICHUNGSFORMEN IN EINEN SPENDER UNTER BILDUNG EINES VIELLAGIGEN STAPELS**
METHOD AND DEVICE FOR INSERTING A PLURALITY OF INDIVIDUAL SHEETLIKE FORMS OF ADMINISTRATION IN A DISPENSER BY FORMING A MULTILAYER PILE
PROCEDE ET DISPOSITIF POUR L'INTRODUCTION D'UNE PLURALITE DE FORMES DE PRESENTATION EN FEUILLES INDIVIDUELLES DANS UN DISTRIBUTEUR, AVEC FORMATION D'UNE PILE MULTICOUCHE

(30) Priorität: 19.02.1998 DE 19806966
(43) Veröffentlichungstag der Anmeldung: 06.12.2000
(60) Teilanmeldung: 03014212.9
(73) Patentinhaber: LTS Lohmann Therapie-Systeme AG, 56626 Andernach (DE)
(72) Erfinder: STEINBORN, Peter, D-56567 Neuwied (DE); DZEKAN, Horst, D-56584 Meinborn (DE); SCHUMANN, Klaus, D-56567 Neuwied (DE); LAUX, Wolfgang, D-65582 Diez (DE); HORSTMANN, Michael, D-56564 Neuwied (DE)
(74) Vertreter: Flaccus, Rolf-Dieter, Dr.
(86) Internationale Anmeldenummer: EP9900584
(87) Internationale Veröffentlichungsnummer: WO99042397

(56) Entgegenhaltungen:
- DE-B- 2 458 148
- US-A- 3 682 468
- US-A- 3 823 934

## Beschreibung

Die Erfindung betrifft ein Verfahren sowie eine Vorrichtung zum Einbringen einer Vielzahl vereinzelter folienförmiger Darreichungsformen in einen Spender unter Bildung eines viellagigen, aus lose und in exakter Ausrichtung aufeinander liegenden Einzellagen bestehenden Stapels zur einzeldosierbaren Entnahme, wobei als Ausgangsmaterial in Rollen aufgewickelte folienförmige Bänder vorliegen. Die Darreichungsformen können für ihre Anwendung therapeutische oder kosmetische bzw. lebensmitteltechnische Produkte enthalten.

Im Mundbereich und auf den Schleimhäuten des Mundes anzuwendende flächige Darreichungsformen sind bekannt. So beschreibt die US 3 444 858 (1969) Medikamentstreifen auf Basis. eines gelatineartigen Materials.

Weiterhin sind Anwendungsvorschläge solcher Folien außerhalb des Arzneimittelbereiches bekannt. In der EP 0 216 762 wird eine wasserlösliche Folie aus Stärke, Gelatine, Glycerin oder Sorbit offenbart, die mittels eines Walzenauftrags beschichtet wird. Dabei wird erwähnt, daß sich solche Dosierformen z.B. auch für chemische Reagenzien, Aromastoffe und dergleichen herstellen lassen.

Auf dem Markt haben sich seit ca. 1995 erhältliche Darreichungsformen im kosmetischen und Süßwaren-Anwendungsbereich mit Einzelspendern aus Kunststoff eingebürgert; erwähnt sei das Produkt der Nisshin, Japan. Die Einzelspender enthalten Stapel aufeinanderliegender Folienabschnitte, die nach Öffnung einer Öffnungslippe die Einzelentnahme des jeweils obersten Folienstückes erlauben.

Die technische Lösung der Erzeugung und Verpackung solcher Stapel aus folienförmigem Ausgangsmaterial stößt jedoch auf Schwierigkeiten; der Literatur sind keine praktikablen Anleitungen zu ihrer Herstellung zu entnehmen. Lediglich US 4 180 558 beschreibt die Stapelung eßbarer Folien, die aber an den Kanten aufeinanderlaminiert sind und sich daher für die vorstehende Anwendungsweise nicht eignen.
Weil solche folienförmigen Darreichungsformen wegen ihres geringen Flächengewichts, beispielsweise zwischen 10 und 50 g/m², zu statischer Aufladung neigen und die Oberflächen zwecks leichter Entnahme verschiebbar ausgeführt sein müssen, gelingt ein exakt positioniertes Schneiden und Aufeinanderlegen aus Folienabschnitten nur mit großen Schwierigkeiten und mit großem Zeitaufwand.

Auch die Beobachtung von Herstelltechniken industrieller Bereiche wie der Papierverarbeitung oder der Kunststoffbeutelverpackung liefert keine praktikablen Hinweise zur Problemlösung. In der US 4 070 014 wird zwar die Bildung von Stapeln aus zwei verschiedenen Papiersorten in teilweise überlappender Anordnung mit Hilfe vakuumansaugender Transferrollen beschrieben. Diese Technik, wie auch die aus der US 5 348 527 bekannte Technik der Papierstaplung beschreibt ein vergleichsweise zeitaufwendiges Verfahren von Einzelschnitten aus einer Rolle und Einzelablage auf einen Stapel.
Die US 4 406 650 beschreibt die Bildung eines in Zick-Zack-Form gefalteten Papierbandes von einer Rolle, aus der ein Stapel durch Beschnitt hervorgeht. Hier liegt ein Nachteil im Verschnitt des Materials. Bei unterschiedlicher Folienstruktur der Ober- und Unterseite oder bei Wellung gelingt es dabei nicht, die Einzelblätter mit ihrer ursprünglichen Oberseite nach oben zu stapeln.

Ausgehend vom vorgenannten Stand der Technik liegt der Erfindung die Aufgabe zugrunde, ein Verfahren zur Herstellung einzeldasierter, als aus lose und in exakter Ausrichtung aufeinander liegenden Einzellagen bestehenden Stapel in einem Spender angeordnete folienförmige Darreichungsformen und eine Vorrichtung zum Einbringen einer Vielzahl von vereinzelten folienförmigen Darreichungsformen für therapeutische, kosmetische oder Lebensmitteltechnische Produkte in einen Spender anzugeben, mit der es unter Vermeidung der vorgenannten Nachteile und Schwierigkeiten gelingt, einzelne dosierbare folienförmige Darreichungsformen präzise und mit hoher Arbeitsgeschwindigkeit in einen Spender unter Bildung eines viellagigen Stapels einzubringen, wobei als Ausgangsmaterial in Rollen aufgewickelte folienförmige Bänder vorliegen.

Diese Aufgabe wird mit der Erfindung bei einem Verfahren der im Oberbegriff von Anspruch 1 genannten Art durch die im Kennzeichnungsteil angegebene Folge von Arbeitsschritten gelöst.
Dabei wird mit Vorteil in überraschend einfacher Arbeitsweise der Stapel durch gleichzeitiges Abwickeln mehrerer Einzelrollen des folienförmigen Zwischenproduktes, Aufeinanderlegen der Einzelbahnen und gemeinsames Querschneiden des entstandenen Mehrfachlaminatstranges und Ablängen der Stapel exakt gebildet und in den Spender eingeschoben. Mit großem Vorteil eignet sich das erfindungsgemäße Verfahren für ein genaues Arbeiten mit großer Geschwindigkeit.

Das hierfür vorgesehene Zwischenprodukt der Schmalrollen besteht beispielsweise aus ca. 20 bis 1000 m langen, aufgerollten Einzelbahnen in Produktbreite, welche durch Rollenschnitt aus ursprünglicher Breitware herstellbar sind.
Im einfachsten Fall wird hierfür eine Anzahl von Schmalrollen, welche der Anzahl von Folienstücken pro Spender entspricht, in der Nähe eines herzustellenden Stapels frei rollbar auf Achsen fixiert.

Die Dreh- und Abwickelrichtung der Rollen ist bevorzugt gleichsinnig, und das Material wird mittels dem Fachmann bekannter Zugeinrichtungen von allen Rollen gemeinsam abgezogen und geführt. Der entstandene Mehrfachlaminatstrang wird unter Kontrolle und Egalisierung der seitlichen Stoßkanten sodann einer Querschnitteinrichtung zugeführt, hinter welcher sich der zu beschickende Spender im geöffneten Zustand befindet.

Die verwendeten Schmalrollen können nahezu beliebige Geometrie aufweisen. Gute Ergebnisse lassen sich mit Rollenbreiten zwischen 5 und 40 mm erzielen. Die Länge des aufgewikkelten Materials sollte mindestens etwa 20 m, bevorzugt über 500 m, pro Rolle betragen, um eine häufige Unterbrechung des Betriebes zu vermeiden. Eine Vorab-Vereinigung von zwei oder mehr Bändern und gleichzeitiges Aufwickeln bzw. Abrollen ist möglich, führt aber häufig zu Schwierigkeiten, wenn viele Schichten gewickelt werden. Es hat sich überraschend als nicht möglich erwiesen, ein aus acht oder mehr solcher Folienschichten entstandenes Laminat in einem gesteuerten Prozeß wieder abzuwickeln, da die auf dem Außenradius liegenden Laminatelemente der Folienstreifen geringfügig länger sind als die inneren Laminatelemente. Beim Abwickeln würde dies z.B. bei mehr als vier Laminatschichten zum Auseinanderlaufen der Bahnen und damit zur Prozessunterbrechung führen.
Aus diesem Grunde wird bevorzugt vom Auf- und Abrollen von Rollen mit wenigen - z.B. bis zu vier - Laminatelementen Gebrauch gemacht.

Die Art des Spenders ist dabei für die Anwendung der vorliegenden Erfindung unerheblich. Spender aus Kunststoff oder Pappe lassen sich problemlos füllen.
Die Öffnungsseite kann sich im Falle rechteckiger Folienabschnitte an der Schmalseite befinden, in welchem Fall die vorgeschnittenen Schmalrollen die Breite der geringen Seitenlänge des Rechtecks aufweisen.
Sofern die Schmalrollen aber in der späteren Längsdimension der Folienabschnitte ausgeführt sind, befindet sich die Öffnungsseite des Spenders auf einer seiner Längsseiten.

Im Falle zweiteiliger Spender würde sinnvollerweise ein z.B. schubladenförmig ausgeführtes Unterteil primär gefüllt und anschließend das Oberteil aufgesetzt werden. Es ist aber auch möglich, halbgeöffnete Spender zu befüllen.
Auch das Befüllen von Siegelbeuteln, die insbesondere im Fall einer "peel"-fähigen Ausführungsform als Spender eingesetzt werden können, ist möglich, indem z.B. der geschnittene Stapel auf die Unterbahn einer zulaufenden siegelfähigen Packstoffbahn aufgelegt und anschließend nach Zufuhr der Packstoffoberbahn der entstandene Beutel zugesiegelt wird.

Einzelheiten, Merkmale und Vorteile der Erfindung ergeben sich beispielhaft aus der nachstehenden Erläuterung eines in der Zeichnung schematisch dargestellten Verfahrensstammbaumes. Dieser zeigt:

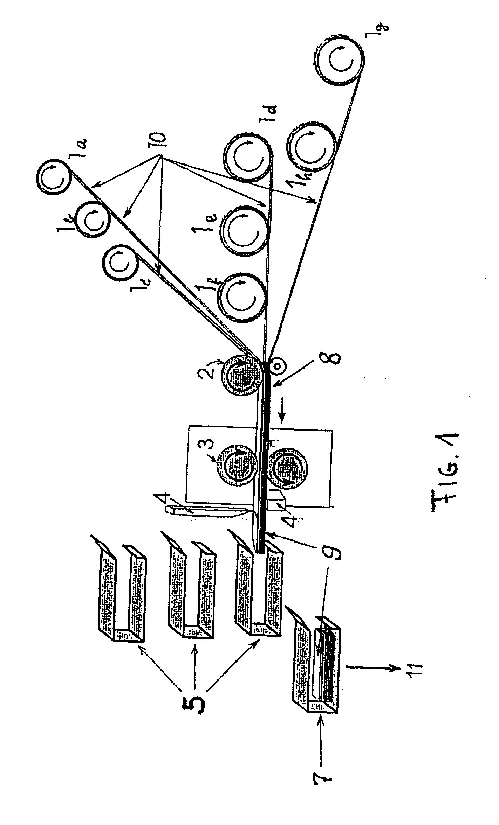

Die Vorrichtung zum Einbringen einer Vielzahl vereinzelter folienförmiger Darreichungsformen in einen Spender unter Bildung eines viellagigen Stapels (9) zur einzeldosierbaren Entnahme umfaßt gemäß Figur 1 eine unbestimmte Anzahl - je nach der Zahl der Lagen - von Rollen (1a bis 1h) mit darauf aufgewickelten wirkstoffhaltigen Bändern (10).
Diese laufen beim Abwickelvorgang mit unterschiedlichen Winkeln zur Horizontalen zu Führungsrollen (2) und zwischen diesen hindurch, wobei die Bänder (10) zu einem viellagigen Strang (8) gebündelt werden. Der Strang (8) wird von einem Paar Förderrollen (3) ergriffen und zu einer Schere (4) gezogen. Am Ende der Transportstrecke angekommen, wird mit der Schere (4) vom Strang (8) jeweils ein viellagiger Stapel (9), bestehend aus lose in exakter Ausrichtung aufeinander liegenden Darreichungsformen, abgetrennt und in einen bereitgestellten Spenderbehälter (5) eingefüllt, nachdem der Spender (5) vorher selbsttätig mit einer (nicht gezeigten) Fördereinrichtung in die Füllposition gebracht wurde. Dabei wird der Spender (5) mit wenigstens einem Stapel (9) beladen. Je nach Material und Aufgabenstellung kann ein Spenderbehälter (5) auch mit mehreren Stapeln (9) sukzessive befüllt werden.
Der beladene Behälter wird dann mit der Fördereinrichtung (11) abtransportiert.

Das Verfahren und die Vorrichtung sind überraschend einfach, kostengünstig erstellbar und lösen in idealer Weise die eingangs gestellte Aufgabe.

## Patentansprüche

1. Herstellungsverfahren für einzeldosierte, als Stapel (9) in einem Spender (5) angeordnete folienförmige Darreichungsformen für therapeutische, kosmetische oder lebensmitteltechnische Produkte, umfassend
- das seitengleiche Aufeinanderlegen der entsprechenden Anzahl von folienförmigen Lagen der von mehreren Rollen (1) zugleich abgezogenen Bänder (10) von Zwischenprodukten zu einem viellagigen Strang (8), unter Verwendung von Führungsmitteln (2),
- das gemeinsame Querschneiden des viellagigen Stranges (8), und
- das Befüllen eines Spenders (5) mit dem viellagigen, aus den lose und in exakter Ausrichtung aufeinander liegenden Einzellagen, welche die Darreichungsformen bilden, bestehenden Stapel (9).

2. Verfahren nach Anspruch 1, **dadurch gekennzeichnet, daß** der Spender (5) nach Befüllen und Verschließen selbsttätig von der Beladestation abgeführt und ein leerer Spender (5) selbsttätig an dessen Stelle zugeführt wird.

3. Verfahren nach Anspruch 1 oder 2, **dadurch gekennzeichnet, daß** auf einer Rolle (1) wenigstens zwei folienförmige Bänder (10) mit gleicher Ausrichtung ihrer Ober- und Unterseiten aufgerollt werden.

4. Verfahren nach einem oder mehreren der Ansprüche 1 bis 3, **dadurch gekennzeichnet, daß** anstelle von Spendern (5) Siegelbeutel zur Aufnahme von Stapeln (9) verwendet werden.

5. Verfahren nach einem oder mehreren der Ansprüche 1 bis 4, **dadurch gekennzeichnet, daß** das Abziehen der Bänder (10) von ihren Rollen (1) optisch überwacht und bei Erscheinen eines Bandendes das Arbeitsverfahren unterbrochen wird.

6. Verfahren nach einem oder mehreren der Ansprüche 1 bis 5, **dadurch gekennzeichnet, daß** in einen Spender (5) wenigstens zwei Stapel (9) eingebracht werden.

7. Vorrichtung zum Einbringen einer Vielzahl von vereinzelten folienförmigen Darreichungsformen für therapeutische, kosmetische oder lebensmitteltechnische Produkte in einen Spender (5) mit
- einer Anzahl von Rollen (1) mit abwickelbaren folienförmigen Bändern (10) von Zwischenprodukten,
- wenigstens einem Führungselement (2) zur Vereinigung der aus unterschiedlichen Richtungen zulaufenden Bänder (10) zu einem viellagigen Strang (8),
- einer Vorzugseinrichtung (3) für den viellagigen Strang (8),
- einer Schneidvorrichtung (4) zum Querschneiden des viellagigen Stranges (8), und
- Transport- und Kontrollmitteln zum Zuführen leerer Spender (5), zum Befüllen eines Spenders (5) mit dem viellagigen, aus lose und in exakter Ausrichtung aufeinander liegenden Einzellagen, welche die Darreichungsformen bilden, bestehenden Stapel (9) und zum Abführen der mit Stapeln (9) befüllten Spender (5).

## Claims

1. Manufacturing process for individually dosed sheet-like administration forms arranged as a stack (9) in a dispenser, for therapeutic, cosmetic or food-technological products, comprising
- equal-sided superimposing of the corresponding number of sheet-like layers of the tapes (10) of intermediate products, which layers have been drawn simultaneously from several rolls (1), to form a multilayered strand (8), by employing guide means (2),
- jointly cross-cutting the multi-layered strand (8), and
- filling a dispenser (5) with the multi-layered stack (9) consisting of the individual layers which are lying loosely one upon the other in exact alignment and which form the administration forms.

2. Process according to Claim 1, **characterized in that** dispenser (5), after filling and closing thereof, is automatically led away from the loading station, and an empty dispenser (5) is automatically fed in its place.

3. Process according to Claim 1 or 2, **characterized in that** on one roll (1) there are wound at least two sheet-like tapes (10) with equal orientation of their top and bottom faces.

4. Process according to one or more of Claims 1 to 3, **characterized in that** instead of dispensers (5) there are used sealing bags for receiving the stacks (9).

5. Process according to one or more of Claims 1 to 4, **characterized in that** the drawing of tapes (10) from their rolls (1) is supervised optically, and that when a tape end appears, the work process is stopped.

6. Process according to one or more of Claims 1 to 5, **characterized in that** into a dispenser (5) there are introduced at least two stacks (9).

7. Device for inserting a plurality of segregated sheet-like administration forms, for therapeutic, cosmetic or food-technological products, into a dispenser (5), comprising
- a number of rolls (1) with sheet-like tapes (10) of intermediate products, which can be unwound,
- at least one guide element (2) for uniting the tapes (10), which are fed from various directions, to form a multilayered strand (8),
- an advancing device (3) for the multi-layered strand (8),
- a cutting device (4) for cross-cutting the multi-layered strand (8), and
- transport and control means for feeding empty dispensers (5), for filling a dispenser (5) with the multilayered stack (9) consisting of individual layers which are lying loosely one upon the other in exact alignment and which form the administration forms, and for leading away the dispensers (5) filled with stacks (9).

## Revendications

1. Procédé de fabrication pour des formes de présentation sous forme de feuilles en doses individuelles disposées en pile (9) dans un distributeur (5) pour des produits thérapeutiques, cosmétiques ou intervenant dans des techniques de traitement de denrées alimentaires, comprenant
- la superposition, en faisant coïncider leurs côtés, du nombre correspondant de couches en forme de feuilles des bandes (10) de produits intermédiaires dévidées simultanément de plusieurs rouleaux (1) afin d'obtenir une nappe multicouche (8), en utilisant des moyens de guidage (2),
- la coupe transversale commune de la nappe multicouche (8) et
- le remplissage d'un distributeur (5) avec la pile multicouche (9) formée des couches individuelles détachées et parfaitement alignées les unes sur les autres qui forment les formes de présentation.

2. Procédé selon la revendication 1, **caractérisé en ce que** le distributeur (5) est éloigné automatiquement de la station de chargement après avoir été rempli et fermé et un distributeur vide (5) prend automatiquement sa place.

3. Procédé selon la revendication 1 ou 2, **caractérisé en ce qu'**au moins deux bandes (10) en forme de feuilles dont le dessus et le dessous ont la même orientation sont enroulées sur un rouleau (1).

4. Procédé selon l'une ou plusieurs des revendications 1 à 3, **caractérisé en ce que** l'on utilise, à la place de distributeurs (5), des sachets à sceller pour recevoir les piles (9).

5. Procédé selon l'une ou plusieurs des revendications 1 à 4, **caractérisé en ce que** le dévidage des bandes (10) de leurs rouleaux (1) est surveillé par des moyens optiques et l'opération est interrompue lorsque la fin d'une bande apparaît.

6. Procédé selon l'une ou plusieurs des revendications 1 à 5, **caractérisé en ce que** l'on introduit au moins deux piles (9) dans un distributeur (5).

7. Dispositif pour l'introduction d'une pluralité de formes de présentation en feuilles individuelles pour des produits thérapeutiques, cosmétiques ou intervenant dans des techniques de traitement de denrées alimentaires dans un distributeur (5), comprenant
- un nombre de rouleaux (1) avec des bandes (10) de produits intermédiaires sous forme de feuilles pouvant être déroulées,
- au moins un élément de guidage (2) pour réunir les bandes (10) arrivant de différentes directions en une nappe multicouche (8),
- un dispositif d'avance (3) pour la nappe multicouche (8),
- un dispositif de coupe (4) pour la coupe transversale de la nappe multicouche (8), et
- des moyens de transport et de contrôle permettant d'amener des distributeurs vides (5), de remplir un distributeur (5) avec la pile multicouche (9) formée de couches individuelles détachées et parfaitement alignées les unes sur les autres qui forment les formes de présentation, et d'éloigner les distributeurs (5) remplis de piles (9).
